## Europäisches Patentamt

## European Patent Office

## Office européen des brevets

(19)

(11) Veröffentlichungsnummer: **0 032 394**
**B1**

(12)

# EUROPÄISCHE PATENTSCHRIFT

(45) Veröffentlichungstag der Patentschrift:
**05.12.84**

(51) Int. Cl.³: **A 61 B 3/10,** G 02 B 5/00

(21) Anmeldenummer: **81100151.0**

(22) Anmeldetag: **10.01.81**

(54) **Entfernungsunabhängiges Ophthalmometer hoher Genauigkeit.**

(30) Priorität: **12.01.80 DE 3000995**

(73) Patentinhaber: **Firma Carl Zeiss, Postfach 1369/1380, D-7082 Oberkochen (DE)**

(43) Veröffentlichungstag der Anmeldung:
**22.07.81 Patentblatt 81/29**

(72) Erfinder: **Müller, Ortwin, Dipl.-Phys., Kolpingstrasse 34, D-7080 Aalen (DE)**
Erfinder: **Schulz, Kurt, Mozartweg 20, D-7082 Oberkochen (DE)**
Erfinder: **Stopar, Viktor, Mozartweg 13, D-7082 Oberkochen (DE)**

(45) Bekanntmachung des Hinweises auf die Patenterteilung:
**05.12.84 Patentblatt 84/49**

(84) Benannte Vertragsstaaten:
**CH FR GB LI**

(56) Entgegenhaltungen:
**DE - B - 2 952 078**
**DE - C - 370 333**
**DE - C - 861 932**
**US - A - 3 290 927**

**Süddeutsche Optikerzeitung (1953) Nr. 1, S. 6-8; Nr. 3, S. 49 u. 50; Nr. 5, S. 112-113**

# Beschreibung

Die Erfindung betrifft ein entfernungsunabhängiges Ophthalmometer mit einem Messwerk zur Bestimmung des Abstandes zweier auf der Hornhaut erzeugter Spiegelbilder von Messmarken mittels zweier übereinanderstehender, mit Hilfe eines auf einer Kurve geführten Hebels gegenläufig schwenkbarer Planglasplatten. Ein derartiges Ophthalmometer ist beispielsweise aus der DE-C-861 932 bekannt.

Bei Ophthalmometern, die den linearen Abstand der Hornhautspiegelbilder messen, liegt die Genauigkeit des erhaltenen Messwertes bei ca. $\pm$ 0,05 mm. Die Entwicklungsarbeiten auf diesem Gebiet zielen auf eine höhere Genauigkeit.

Einen Überblick über die Probleme der Ophthalmometrie und der gebräuchlichen Ophthalmometer gibt der Aufsatz von H. Littmann in der Süddeutschen Optikerzeitung II/53. Ein aus der Analyse der dort beschriebenen Probleme der Ophthalmometrie entstandenes Ophthalmometer wird von H. Littmann in der Süddeutschen Optikerzeitung V/53 beschrieben.

Der vorliegenden Erfindung liegt die Aufgabe zugrunde, ein neues Ophthalmometer mit höherer Messgenauigkeit zu entwickeln, das auch für die Kontaktlinsenanpassung geeignet ist. Diese Aufgabe wird erfindungsgemäss dadurch gelöst, dass in Weiterentwicklung des Ophthalmometers nach Helmholz und der in Abbildung 5 des Aufsatzes von H. Littmann in der Süddeutschen Optikerzeitung II/53 gezeigten Variante die Glasplatten fest in Walzen gelagert sind, die über Kugellager in Prismenlagern drehbar gelagert sind, dass auf die Walzen Zugfedern wirken, dass ausserdem die Hebel für die Schwenkbewegung der Glasplatten auf der Kurve mittels eines Zwischenglieds geführt sind und dass zum Kompensieren von Fertigungstoleranzen auf dem Hebel ein Kugellager einstellbar gelagert ist und das Zwischenglied mit einer verstellbaren Membran verbunden ist.

Die mit der Erfindung erzielten Vorteile liegen in der erreichbaren Messgenauigkeit von $\pm$ 0,015 mm.

Ein Ausführungsbeispiel der Erfindung ist in der Zeichnung dargestellt und wird im folgenden näher beschrieben. Es zeigen

Fig. 1 eine Prinzipskizze der Erfindung;

Fig. 2 einen Querschnitt durch das erfindungsgemässe Messwerk;

Fig. 3 eine Seitenansicht des in Figur 2 dargestellten Messwerks;

Fig. 4 einen Längsschnitt durch das in den Fig. 1 und 2 dargestellte Messwerk.

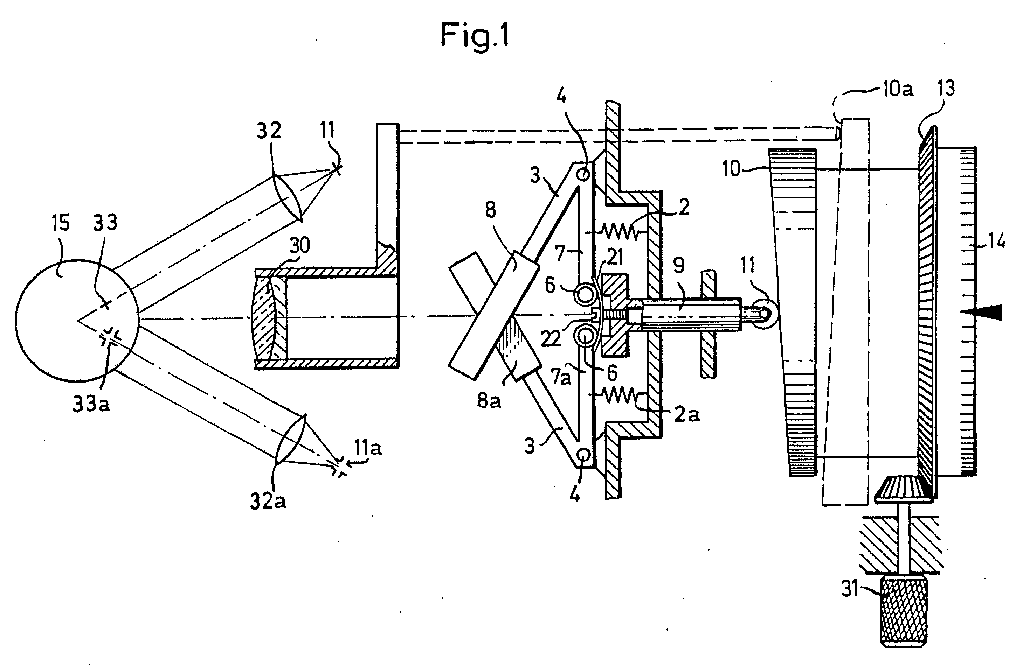

In der in Fig. 1 dargestellten Prinzipskizze ist das Patientenauge mit 15 bezeichnet. Auf der Hornhaut des Patientenauges werden die Messmarken 11, 11a über Kollimatoren 32, 32a als Spiegelbilder 33, 33a abgebildet. Der lineare Abstand dieser Spiegelbilder wird entsprechend der Helmholtzschen Methode mit Hilfe zweier übereinanderstehender, gegenläufig schwenkbarer Planglasplatten 8, 8a gemessen. Aus der Prinzipskizze geht hervor, dass durch Drehen am Bedienknopf 31 das Antriebsrad 13 und mit diesem der Skalenkreis 14 und die mit ihm verbundene Kurve 10 bewegt wird. Über das Kugellager 11, und das Zwischenglied 9 und die Hebel 7, 7a werden die über Federn 2, 2a spielfrei gelagerten Glasplatten 8, 8a gedreht. Die über die Kurve 10a gesteuerte Linse 30 dient dem Astigmatismusausgleich.

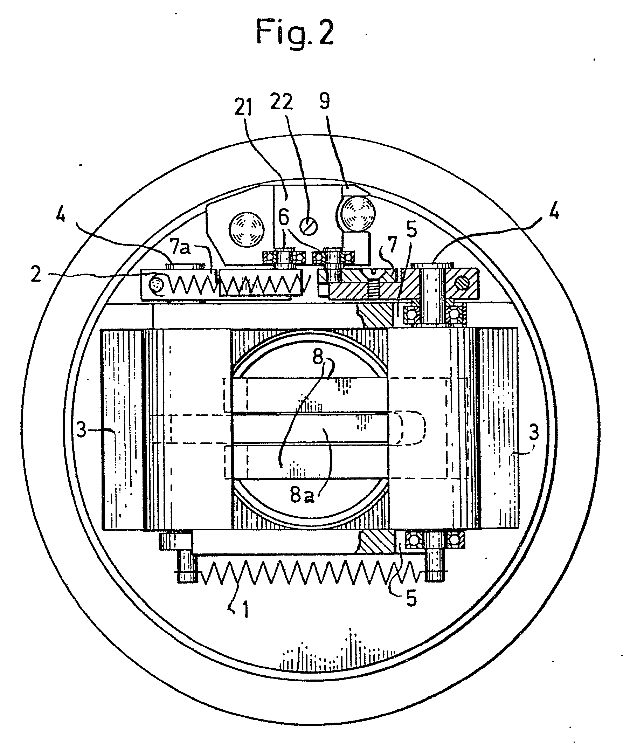

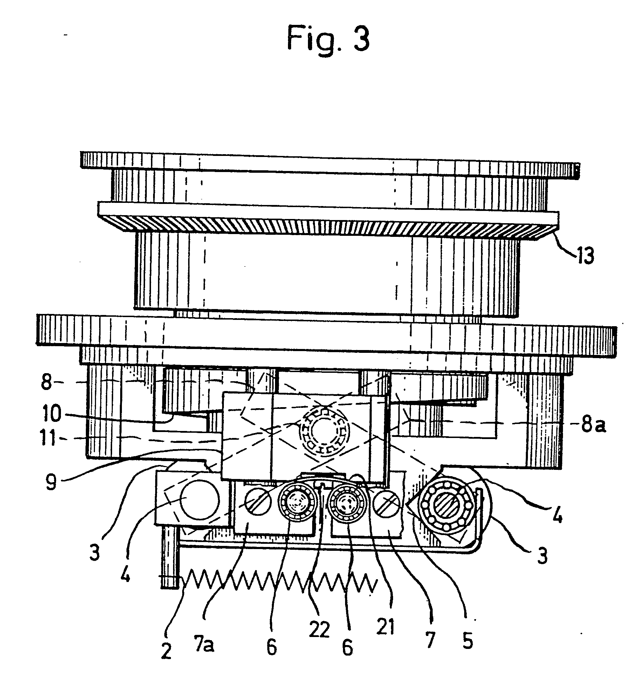

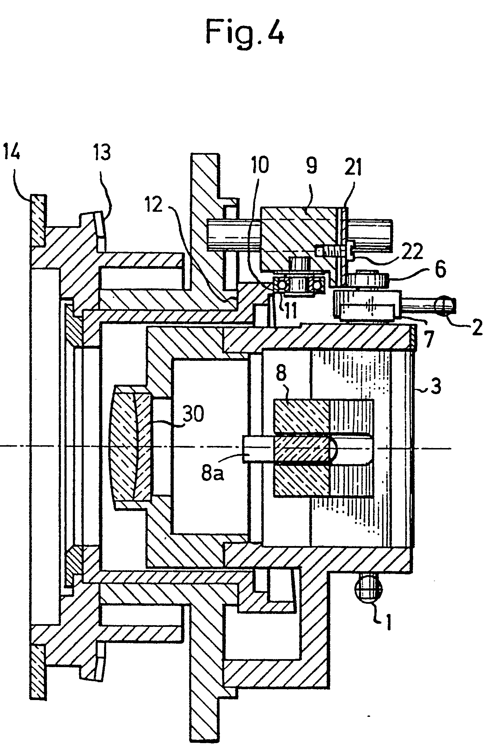

Die spielfreie Lagerung der Glasplatten 8, 8a sowie ein Justierelement zur Kompensierung der Fertigungstoleranzen ist in den Fig. 2, 3 und 4 dargestellt. Die Glasplatten 8, 8a sind in Walzen 3 fest gelagert, diese wiederum sind über Kugellager 4 in den Prismenlagern 5 drehbar gelagert. Das Drehen der Glasplatten wird über Hebel 7 bewirkt, die von der Kurve 10 mittels Zwischenglied 9 angesteuert werden. Mit den Federn 1, 2 wird eine spielfreie Lagerung erreicht. Die Walzen 3 sind infolge der Zugkraft der Federn 1, 2 in den Prismenlagern 5 spielfrei und trotzdem leicht drehbar gelagert. Da bei der Zugfeder 2 der Angriffspunkt ausserhalb des Mittelpunktes der Drehachse angeordnet ist, entsteht ein zusätzliches Drehmoment, das über die Hebel 7 und das Kugellager 6 eine Kraft in Achsrichtung auf das Zwischenglied 9 ausübt, das über das Kugellager 11 die Kurve 10 gegen die Fläche 12 drückt. Dadurch ist auch diese für die Messung wichtige Fläche spielfrei. Über die mittels Stellschraube 22 verstellbare Membran 21 ist eine Justiermöglichkeit für das Zwischenglied 9 gegeben. Ausserdem kann das Kugellager 6 auf dem Hebel 7 einstellbar gelagert sein. In diesem Fall ist es möglich, die Hebellänge des Hebels zu verändern. Dadurch ist die Drehbewegung der Glasplatten 8, 8a bei gegebener Plattendicke und gegebener Kurvensteigung 10 bei einer bestimmten Drehung des Skalenkreises 14 (und des damit verbundenen Antriebszahnrads 13 und der Kurve 10) einstellbar.

## Patentanspruch

1. Entfernungsunabhängiges Ophthalmometer mit einem Messwerk (13, 14) zur Bestimmung des Abstandes zweier auf der Hornhaut erzeugter Spiegelbilder von Messmarken (11, 11a) mittels zweier übereinanderstehender, mit Hilfe eines auf einer Kurve (10) geführten Hebels (7, 7a) gegenläufig schwenkbarer Planglasplatten (8, 8a) dadurch gekennzeichnet, dass die Planglasplatten (8, 8a) fest in Walzen (3) gelagert sind, die über Kugellager (4) in Prismenlagern (5) drehbar gelagert sind, dass auf die Walzen (3) Zugfedern (1, 2) wirken, dass ausserdem die Hebel (7) für die Schwenkbewegung der Planglasplatten (8, 8a) auf der Kurve (10) mittels eines Zwischengliedes (9) geführt sind und dass zum Kompensieren von Fertigungstoleranzen auf dem Hebel (7) ein Kugellager (6) einstellbar gelagert ist und das Zwischenglied (9) mit einer verstellbaren Membran (21) verbunden ist.

## Claim

An opthalmometer which is independent of distance having a measurement mechanism (13, 14) for determining the distance between two mirror images (on the cornea) of measurement marks (11, 11a) by means of two flat glass plates (8, 8a) which are arranged one above the other and which are swingable in opposite direction by means of a lever (7, 7a) which is guided on a cam (10), characterized in that the glass plates (8, 8a) are supported fixed on hubs (3) which are pivotally supported via ball-bearings (4) in vee supports (5), that tension springs (1, 2) act on the rollers (3), that the levers (7, 7a) for the swinging movement of the glass plates (8, 8a) are guided on the cam (10) by an intermediate member (9) and that for the compensation of manufacturing tolerances a ball-bearing (6) is adjustably supported on the lever (7) and the intermediate member (9) is connected with an adjustable diaphragm (21).

**Revendication**

Ophthalmomètre indépendant de la distance, avec un système de mesure (13, 14) destiné à déterminer la distance de deux images symétriques de repères de mesure (11, 11a) obtenues sur la cornée, à l'aide de deux plaques de verre plan (8, 8a), disposées l'une au-dessus de l'autre, pouvant subir un pivotement opposé à l'aide d'un levier (7, 7a) guidé sur une came (10), caractérisé en ce que les plaques de verre plan (8, 8a) sont logées à demeure dans des cylindres (3), qui sont logés d'une manière rotative dans des logements prismatiques (5) par l'intermédiaire de roulements à billes (4); que des ressorts de traction (1, 2) agissent sur les cylindres (3); que, par ailleurs, les leviers (7) servant au mouvement de pivotement des plaques de verre plan (8, 8a) sont guidés sur la came (10) à l'aide d'un organe intermédiaire (9); et que, pour la compensation des tolérances de finition, un roulement à billes (6) est logé d'une manière réglable sur le levier (7), et que l'organe intermédiaire (9) est relié à une membrane mobile (21).

Fig.1

# Fig. 2

# Fig. 3

# Fig. 4